(19) Europäisches Patentamt
European Patent Office
Office européen des brevets

(11) Veröffentlichungsnummer : **0 073 442**
**B1**

(12) # EUROPÄISCHE PATENTSCHRIFT

(45) Veröffentlichungstag der Patentschrift : **13.03.85**

(51) Int. Cl.⁴ : **C 07 C125/063**, C 07 C155/02

(21) Anmeldenummer : **82107701.3**

(22) Anmeldetag : **23.08.82**

(54) **Neue N-Alkoxycarbonyl- und N-Alkoxythiocarbonylamidine und Verfahren zu ihrer Herstellung.**

(30) Priorität : **28.08.81 DE 3134144**

(43) Veröffentlichungstag der Anmeldung : **09.03.83 Patentblatt 83/10**

(45) Bekanntmachung des Hinweises auf die Patenterteilung : **13.03.85 Patentblatt 85/11**

(84) Benannte Vertragsstaaten : **BE CH DE FR GB LI**

(56) Entgegenhaltungen :
**CHEMICAL ABSTRACTS, Band 83, Nr. 1, 7. Juli 1975, Seite 760, Nr. 9148a, Columbus, Ohio, USA V.P. KUKHAR et al.: "Polychloroalkylamines. VIII. Reaction of trichloromethyl dialkylamines with N,N-dichlorourethane and with the methyl ester of N-chloroiminobenzoic acid"**

(73) Patentinhaber : **BASF Aktiengesellschaft
Carl-Bosch-Strasse 38
D-6700 Ludwigshafen (DE)**

(72) Erfinder : **Hamprecht, Gerhard, Dr.
Rote-Turm-Strasse 28
D-6940 Weinheim (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 073 442 B1

Jouve, 18, rue St-Denis, 75001 Paris, France

**Beschreibung**

Die Erfindung betrifft die neuen N-Alkoxycarbonyl- und N-Alkoxythiocarbonylamidine und ein Verfahren zur Herstellung von N-Alkoxycarbonyl- und N-Alkoxythiocarbonylamidinen durch Umsetzung von Amidinen mit Estern der Chlorkohlensäure oder Chlorthiokohlensäure in Gegenwart eines anorganischen Säureakzeptors und eines 2-phasigen, wäßrig-organischen Lösungsmittelgemisches bei einer Temperatur von 0 bis 60 °C.

Es ist bekannt, Ethoxycarbonylbenzamidin durch Umsetzung von freiem Benzamidin mit Chlorkohlensäureethylester in wäßrigalkalischer Lösung herzustellen (Ber. *23*, 2919 (1890)). Hierbei muß Benzamidin zunächst mit der Hälfte der theoretischen Menge Chlorkohlensäureester umgesetzt werden ; aus dem Filtrat kann durch nochmalige Zugabe von Natronlauge und dann von Chlorkohlensäureester eine weitere Menge an Endprodukt erhalten werden, wobei keine Ausbeuteangaben gemacht werden. Auf Alkylamidine kann dieses Verfahren nicht angewandt werden, da sie sich in wäßriger Lösung sehr rasch zersetzen (S. Patai, The chemistry of amidines and imidates, J. Wiley & Sons, 1975, Seite 350), während Phenylsubstituenten stabilisierend wirken.

Bei Verwendung organischer Lösungsmittel und Basen erhielten H. L. Wheeler et al. im Falle des N-Phenyl-phenylacetamidins trotz stöchiometrischen Einsatzes von Pyridin und Benzoylchlorid nur ein diacyliertes Produkt (J. Amer. Chem. Soc. *25*, 796 (1903)).

An gleicher Stelle (S. 795) wird berichtet, daß die Acylierung unbefriedigende Ergebnisse lieferte, da die sich bildenden Zersetzungsprodukte die Isolierung der Acylamidine erschwerten. Als relativ beste Methode wurde die Umsetzung von freiem Amidin mit Benzoylchlorid in Ether ohne Anwesenheit von Base hervorgehoben ; hierbei geht jedoch die Hälfte des Amidins verloren ; ferner müssen die Endprodukte durch Umkristallisation gereinigt werden.

S. P. Joshi et al. (J. Chem. Soc. *1936*, Seiten 793-797) beschreiben die Kondensation von Chlorkohlensäureethylester mit N,N'-Di-m-nitrophenylbenzamidin in Benzol in Gegenwart von Natriumhydrogencarbonat während 12 Stunden, wobei jedoch nur in 48,5 % Ausbeute ein monoacyliertes Rohprodukt erhalten wird, das ebenfalls umkristallisiert werden muß.

Im Hinblick auf die Reaktionsbedingungen sind alle bekannten Verfahren bezüglich einfacher und wirtschaftlicher Arbeitsweise, gerade auch im industriellen Maßstab, unbefriedigend.

Es wurde nun gefunden, daß man N-Alkoxycarbonyl- oder N-Alkoxythiocarbonylamidine der Formel

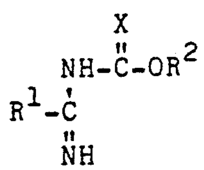

(I)

worin $R^1$ ein Wasserstoffatom, einen aliphatischen, cycloaliphatischen oder araliphatischen Rest bedeutet, $R^2$ einen aliphatischen Rest bezeichnet und X für ein Sauerstoffatom oder ein Schwefelatom steht, vorteilhaft erhält, wenn man Amidine der Formel

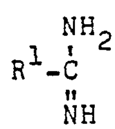

(II)

worin $R^1$ die vorgenannte Bedeutung besitzt, oder ihre Amidinsalze mit Estern der Chlorkohlensäure oder Chlorthiokohlensäure der Formel

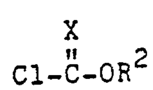

(III)

worin $R^2$ und X die vorgenannte Bedeutung besitzen, in Gegenwart eines anorganischen Säureakzeptors und eines 2-phasigen, wäßrig-organischen Lösungsmittelgemisches bei einer Temperatur von 0 bis 60 °C umsetzt.

Weiterhin wurden die neuen N-Alkoxycarbonyl- oder N-Alkoxythiocarbonylamidine der Formel

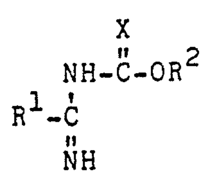

(I)

worin R[1] ein Wasserstoffatom, einen aliphatischen, cycloaliphatischen oder araliphatischen Rest bedeutet, R[2] einen aliphatischen Rest bezeichnet und X für ein Sauerstoffatom oder ein Schwefelatom steht, gefunden.

Verwendet man Propionamidin und Chlorkohlensäureethylester als Ausgangsstoffe, so kann der Reaktionsablauf durch folgendes Formelschema wiedergegeben werden :

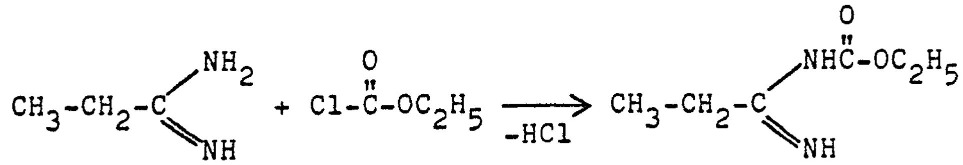

Im Hinblick auf den Stand der Technik liefert das erfindungsgemäße Verfahren auf einfacherem und wirtschaftlicherem Wege Alkoxycarbonyl- und Alkoxythiocarbonylamidine in vergleichsweise besserer Ausbeute und Reinheit. Auch tritt unter den erfindungsgemäßen Bedingungen trotz der Anwesenheit von Wasser im wesentlichen keine Hydrolyse der Ausgangsstoffe II oder III auf. Ebenso wird eine Diacylierung unter den erfindungsgemäßen Bedingungen nicht beobachtet. Das Verfahren kann gerade mit unsubstituierten, instabilen Alkylamidinen durchgeführt werden. Alle diese vorteilhaften Eigenschaften sind nach dem Stand der Technik überraschend.

Bevorzugte Ausgangsstoffe II und III und dementsprechend bevorzugte Endstoffe I sind solche, in deren Formeln R[1] Wasserstoff, einen unsubstituierten oder gegebenenfalls durch Halogenatome, insbesondere Bromatome oder Chloratome, Alkoxygruppen, Alkylmercaptogruppen mit jeweils 1 bis 4 Kohlenstoffatomen je Alkylgruppe, substituierten Alkylrest mit 1 bis 10, insbesondere 1 bis 6 Kohlenstoffatomen, einen unsubstituierten oder gegebenenfalls durch Halogenatome, insbesondere Bromatome oder Chloratome, Alkoxygruppen, Alkylmercaptogruppen mit jeweils 1 bis 4 Kohlenstoffatomen je Alkylgruppe substituierten Alkenylrest oder Alkinylrest mit 2 bis 10, insbesondere 2 bis 6 Kohlenstoffatomen, einen Cycloalkylrest mit 3 bis 7 Kohlenstoffatomen, einen unsubstituierten oder gegebenenfalls durch Halogenatome, insbesondere Bromatome oder Chloratome, substituierten Aralkylrest mit 7 bis 12 Kohlenstoffatomen bedeutet, R[2] einen Alkylrest mit 1 bis 5 Kohlenstoffatomen bezeichnet und X für ein Sauerstoffatom oder Schwefelatom steht. Die aliphatischen Reste können geradkettig oder verzweigt sein. Die cycloaliphatischen Reste können cycloaliphatische Ringkörper oder cycloaliphatische Ringkörper, die durch eine aliphatische Kette mit dem Amidinrest substituiert sind, bedeuten. Die vorgenannten Reste können noch durch unter den Reaktionsbedingungen inerte Gruppen, z. B. Alkylgruppen oder Alkoxygruppen oder Alkylmercaptogruppen mit 1 bis 4 Kohlenstoffatomen substituiert sein. Die Ausgangsstoffe II und III können in stöchiometrischer Menge oder im Überschuß der einen Komponente von der anderen, vorzugsweise in einem Verhältnis 0,8 bis 1,2, insbesondere 1 bis 1,1 Mol Ausgangsstoff III je Mol Ausgangsstoff II, umgesetzt werden.

Beispielsweise kommen folgende Amidine II in Betracht : Formamidin, Acetamidin, Propionamidin, Butyramidin, Isobutyramidin, Valeramidin, Isovaleramidin, sek.-Valeramidin, Pivalinamidin, Capronamidin, α-Methylvaleramidin, Önanthamidin, Pelargonamidin, Caprinamidin, Propenamidin, Propinamidin,

Buten-2-amidin-4 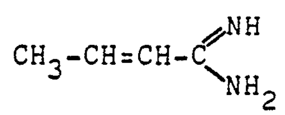 , Buten-1-amidin-4,

Butin-1-amidin-4, Butin-2-amidin-4, Cyclopropylmethanamidin, Cyclopentylmethanamidin, Cyclohexylmethanamidin, Cycloheptylmethanamidin, Chloracetamidin, α-Chlorpropionamidin, β-Chlorpropionamidin, Methoxyacetamidin, α-Methoxypropionamidin, β-Methoxypropionamidin, α-Methylmercaptopropionamidin, β-Methylmercaptopropionamidin, Phenylethanamidin, 2-Chlorphenylethanamidin, 3-Chlorphenylethanamidin, 4-Chlorphenylethanamidin, 3,4-Dichlorphenylethanamidin, 2,4-Dichlorphenylethanamidin.

Ausgangsstoffe der Formel III sind beispielsweise : Chlorameisensäuremethylester, Chlorameisensäureethylester, Chlorameisensäurepropylester, Chlorameisensäureisopropylester, Thiokohlensäure-O-methylesterchlorid, Thiokohlensäure-O-ethylesterchlorid.

Die Reaktion wird bei einer Temperatur von 0 bis 60 °C, vorzugsweise 10 bis 30 °C, drucklos oder unter Druck, kontinuierlich oder diskontinuierlich durchgeführt.

Man führt die Reaktion zweckmäßig in einer vorgelegten Menge an Ausgangsstoff II oder dessen Salz und einem unter den Reaktionsbedingungen inerten, organischen Lösungsmittel in Anwesenheit von Wasser durch. Als organische Lösungsmittelanteile eignen sich besonders chlorierte aliphatische Kohlenwasserstoffe wie Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen ; chlorierte aromatische Kohlenwasserstoffe wie Chlorbenzol, Fluorbenzol, Brombenzol, Jodbenzol, o-, p- und m-Dichlorbenzol, o-, p-, m-Dibrombenzol, o-, m-, p-Chlortoluol, 1,2,4-Trichlorbenzol ; Ether, z. B. Ethylpropylether, Methyl- tert.-butylether, n-Butylethylether, Di-n-butylether, Diisobutylether, Diisoamylether, Diisopropylether, Anisol, Phenetol, Cyclohexylmethylether, Diethylether, Thioanisol, β,β'-Dichlordiethylether; Nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril, aliphatische oder cycloaliphatische Kohlenwasserstoffe, z. B. Heptan, Pinan, Nonan, o-, m-, p-Cymol, Benzinfraktionen innerhalb eines Siedepunktintervalls von 70 bis 190 °C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester z. B. Ethylacetat, Acetessigester, Isobutylacetat; Amide, z. B. 2-Ethylhexansäuredimethylamid, aromatische Kohlenwasserstoffe, z. B. Toluol, Xylol und entsprechende Gemische. Zweckmäßig verwendet man das Lösungsmittelgemisch in einer Menge von 100 bis 2 000 Gew.-%, vorzugsweise 200 bis 1 000 Gew.-%, bezogen, auf Ausgangsstoff II, wobei der Anteil an Wasser an der gesamten Lösungsmittelmenge zwischen 10 bis 50 Gew.-%, vorzugsweise 20 bis 40 Gew.-%, liegt.

Als Säureakzeptoren können vorzugsweise Zink-, Alkali- und Erdalkaliverbindungen, zweckmäßig Hydroxide, Carbonate, Bicarbonate, Oxide, Azetate, Formiate, sowie entsprechende Gemische verwendet werden. Es kommen z. B. als basische Verbindungen in Frage: Kaliumhydroxid, Natriumhydroxid, Natriumcarbonat, Kaliumcarbonat, Lithiumhydroxid, Lithiumcarbonat; Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Magnesiumacetat, Zinkhydroxid, Zinkoxid, Zinkcarbonat, Zinkbicarbonat, Zinkacetat, Natriumformiat, Natriumacetat.

Das Säurebindemittel wird zweckmäßig in äquivalenten Mengen oder in einem Überschuß von bis zu 20 Gew.-%, bezogen auf Amidin der Formel II, eingesetzt. In einer bevorzugten Form des erfindungsgemäßen Verfahrens werden jedoch direkt die Salze der Amidine eingesetzt, wobei zweckmäßig dann das Säurebindemittel in einem Überschuß von bis zu 120 Gew.-%, bezogen auf Amidin II, eingesetzt wird. In diesem Fall wird das zur Reaktion gelangende Amidin der Formel II direkt unter den Reaktionsbedingungen freigesetzt.

Zweckmäßig wird das Verfahren so durchgeführt, daß man über zwei Zuführungen den Ausgangsstoff III und die äquivalente Menge an wäßrigem Säureakzeptor bei der Reaktionstemperatur zu einer ungefähr äquivalenten Menge an Amidin der Formel II in einem inerten, organischen Lösungsmittel zulaufen läßt. Im Falle der bevorzugten Verwendung eines Amidinsalzes setzt man zweckmäßig die doppelt äquivalente Menge an Säureakzeptor je Mol Ausgangsstoff II zu.

Man kann jedoch auch ein Gemisch des Ausgangsstoffes II oder seines Salzes in einem inerten organischen Lösungsmittel oder in Wasser und über eine zweite Zuführung den wäßrigen Säureakzeptor zu einer Lösung des Ausgangsstoffs III in einem inerten organischen Lösungsmittel bei 0 bis 60 °C, vorzugsweise 10 bis 30 °C, zulaufen lassen. Zur Beendigung der Umsetzung rührt man noch 2 Minuten bis 6 Stunden bei 0 bis 60 °C, vorzugsweise 10 bis 30 °C, nach.

Bei der Aufarbeitung saugt man in dem Reaktionsgemisch gegebenenfalls ausgefallenes Neutralisationssalz ab und trennt in dem Filtrat die organische Phase ab. Gegebenenfalls wird die organische Phase nochmals mit Wasser extrahiert und dann getrocknet oder direkt eingeengt. Die gewünschten Endstoffe fallen hierbei in reiner Form an, gegebenenfalls können sie durch Umkristallisieren gereinigt werden. Da die anfallenden Rohprodukte bereits sehr rein sind, kann man jedoch in den meisten Fällen auf ihre Isolierung verzichten und direkt ihre Lösung für weitere Umsetzungen heranziehen.

Als im Gleichgewicht befindliche Verbindungen sind neben den Endstoffen der Formel I auch ihre tautomeren Formen der Formel Ia Gegenstand der Erfindung.

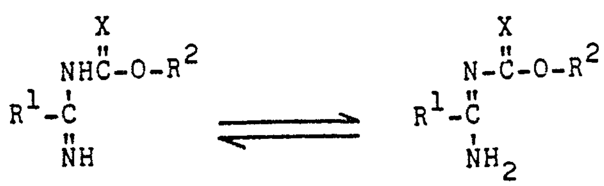

(I) (Ia)

Die so erfindungsgemäß herstellbaren neuen Endstoffe sind wertvolle Ausgangsstoffe für Farbstoffe, Pharmazeutika und Pflanzenschutzmittel und eignen sich insbesondere zur Herstellung neuer, selektiv wirkender Herbizide. Beispielsweise lassen sie sich mittels Aminosulfonylchloriden und Ringschluß in substituierte 2H-1,2,4,6-Thiatriazin(3) on-1,1-dioxide umwandeln. Diese wirken bereits selbst herbizid oder lassen sich mit halogenierenden Agentien in 3-Halogen-2H-1,2,4,6-thiatriazin-1,1-dioxide umwandeln. Auch diese wirken herbizid oder lassen sich in neue, substituierte 2H-1,2,4,6-Thiatriazin-1,1-dioxide überführen, die ausgezeichnet herbizid wirken.

(Siehe Schema Seite 5 f.)

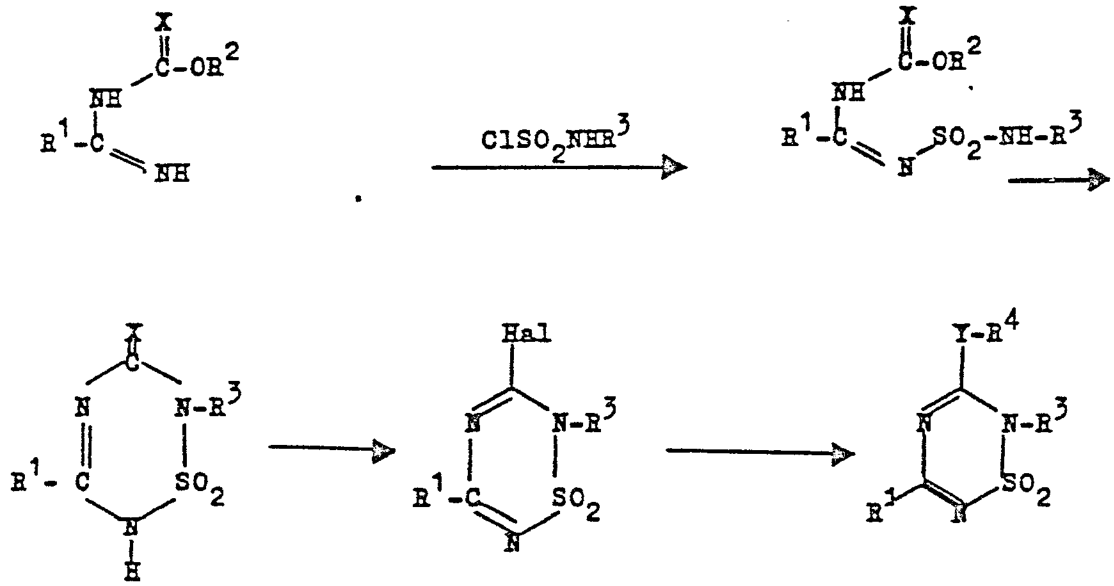


Die in den folgenden Beispielen aufgeführten Teile bedeuten Gewichtsteile.

Beispiel 1

a) Vergleichsbeispiel

80 Teile 50 %ige Natronlauge werden bei 25 °C zu einer Lösung von 94,5 Teilen Acetamidiniumchlorid in 400 Teilen Wasser unter Rühren zugegeben. Anschließend werden 47,3 Teile Chlorameisensäuremethylester bei gleicher Temperatur unter Rühren innerhalb 10 Minuten zugeführt und 10 Minuten nachgerührt. Unter gleichen Bedingungen werden dann nochmals 80 Teile 50 %ige Natronlauge und 47,3 Teile Chlorameisensäuremethylester zugegeben. Nach 10 Minuten Nachrühren wird das Reaktionsgemisch dreimal mit je 130 Teilen Methylenchlorid extrahiert. Aus der organischen Phase werden nach dem Einengen 13 Teile (= 11 % der Theorie) N-Methoxycarbonylacetamidin mit Fp 105-110 °C erhalten.

b) (Erfindungsgemäß)

23 Teile Chlorameisensäuremethylester und 36,5 Teile 50 %ige Natronlauge werden innerhalb 20 Minuten unter Rühren bei 18 bis 20 °C zu einer Mischung von 20 Teilen Acetamidinhydrochlorid in 120 Teilen Methyl-tert.-butylether und 20 Teilen Wasser gegeben. Nach 20 Minuten Rühren bei 20 °C werden die Phasen getrennt und die wäßrige Phase noch zweimal mit je 40 Teilen Methyl-tert.-butylether extrahiert. Aus dem organischen Extrakt werden nach dem Einengen im Vakuum 15 Teile (= 60,8 % der Theorie) N-Methoxycarbonylacetamidin mit Fp 120-125 °C erhalten.

Beispiel 2

Über zwei Zuführungen werden 222 Teile Chlorameisensäuremethylester und 377 Teile 50 %ige Natronlauge unter Rühren bei 10 bis 15 °C innerhalb 25 Minuten zu einer Mischung von 286 Teilen Butyramidinhydrochlorid in 1 300 Teilen Methylenchlorid und 500 Teilen Wasser gegeben. Nach 2 Stunden Rühren bei Raumtemperatur wird die organische Phase abgetrennt und am Rotationsverdampfer eingeengt. Es werden 320 Teile (95 % der Theorie) N-Methoxy-carbonylbutyramidin mit $n_D^{25}$ = 1,476 3 erhalten.

Beispiel 3

a) Über zwei Zuführungen werden 298,6 Teile Chlorameisensäuremethylester und 472,8 Teile 50 %ige Natronlauge unter Rühren bei 20 bis 25 °C innerhalb 25 Minuten zu einer Mischung von 298 Teilen Propionamidinhydrochlorid in 1 300 Teilen Methylenchlorid und 200 Teilen Wasser gegeben. Nach drei Stunden Rühren bei 25 °C wird das Reaktionsgemisch mit 300 Teilen Wasser verdünnt und die Phasen getrennt. Die wäßrige Phase wird einmal mit 200 Teilen Methylchlorid extrahiert. Aus dem organischen Extrakt werden nach dem Abziehen des Lösungsmittels 325 Teile (= 91 % der Theorie) N-Methoxycarbonylpropionamidin mit Fp 87-93 °C erhalten.

b) Unter gleichen Reaktionsbedingungen, jedoch unter Verwendung von 1 300 Teilen Essigester und

einer Nachrührzeit von 30 Minuten, wird der gleiche Endstoff in gleicher Ausbeute und Reinheit gewonnen.

Beispiel 4

165 Teile Chlorameisensäuremethylester und 141,8 Teile Natriumhydroxid in 590 Teilen Wasser werden über zwei Zuführungen unter Rühren bei 25 bis 30 °C zu 221 Teilen 2-Methylpropionamidinhydrochlorid in 1 000 Teilen Diethylether innerhalb 40 Minuten gegeben. Nach einer Stunde Rühren bei Raumtemperatur wird die organische Phase abgetrennt. Die wäßrige Phase wird nochmals mit 200 Teilen Diethylether extrahiert. Aus den vereinigten organischen Auszügen werden nach dem Entfernen des Lösungsmittels 223 Teile (86 % der Theorie) N-Methoxycarbonyl-2-methylpropionamidin mit Fp 127-130 °C erhalten.

Beispiel 5

137,2 Teile Chlorameisensäuremethylester und eine Lösung von 162,7 Teilen Kaliumhydroxid in 180 Teilen Wasser werden über zwei Zuführungen bei 10 bis 15 °C innerhalb 35 Minuten zu einer Mischung von 246 Teilen Benzylmethanamidinhydrochlorid in 530 Teilen 1,2-Dichlorethan und 85 Teilen Wasser gegeben. Nach 20 Minuten Rühren bei 25 °C wird die organische Phase abgetrennt und die wäßrige Phase noch zweimal mit 1,2-Dichlorethan extrahiert. Die organischen Extrakte werden eingeengt, wobei 259,8 Teile (93 % der Theorie) N-Methoxycarbonylbenzylmethanamidin mit $n_D^{25} = 1,524\,7$ erhalten werden.

Beispiel 6

78,8 Teile Chlorameisensäuremethylester und eine Mischung von 46,8 Teilen Kaliumhydroxid in 215 Teilen Wasser werden unter Rühren über zwei Zuführungen bei 0 bis 10 °C zu einer Mischung von 113,8 Teilen Isovaleramidinhydrochlorid in 530 Teilen Methylenchlorid innerhalb 25 Minuten gegeben. Nach 30 Minuten Rühren bei 25 °C werden die Phasen getrennt und die wäßrige Phase einmal mit Methylenchlorid extrahiert. Nach dem Entfernen des Lösungsmittels aus dem organischen Extrakt werden 123 Teile (93,3 % der Theorie) N-Methoxycarbonylisovaleramidin mit Fp 82-86 °C erhalten.

Beispiel 7

Über zwei Zuführungen werden 123 Teile Chlorameisensäureethylester und 170,4 Teile 50 %ige Natronlauge unter Rühren bei 10 bis 15 °C innerhalb 30 Minuten zu einer Mischung von 94.6 Teilen Acetamidinhydrochlorid in 750 Teilen Methylenchlorid und 250 Teilen Wasser gegeben. Nach 15 Minuten Rühren bei 25 °C werden die Phasen getrennt. Die wäßrige Phase wird noch zweimal mit je 100 Teilen Methylenchlorid extrahiert. Nach dem Entfernen des Lösungsmittels aus dem organischen Extrakt werden 93,4 Teile (72 % der Theorie) N-Ethoxycarbonylacetamidin mit Fp 111-114 °C erhalten.

Analog der Arbeitsweise in Beispiel 2 werden folgende Beispiele erhalten :

(Siehe Tabelle Seite 7 f.)

| Beispiel | Teile | $R^1-C(=NH)-NH_2 \cdot HCl$ $R^1$ | Teile $Cl-C(=O)OCH_3$ | Teile NaOH 50 % | Teile | $R^1-C(=N-CO_2CH_3)-NH_2$ $R^1$ | a Fp/ b $n_D^{25}$ | Ausbeute in % der Theorie |
|---|---|---|---|---|---|---|---|---|
| 8 | 64,5 | $Cl-CH_2$ | 53,5 | 92,8 | 45,1 | $Cl-CH_2$ | a) 94-97°C | 60 |
| 9 | 67 | $CH_3-O-CH_2CH_2-$ | 45,8 | 78 | 59 | $CH_3-O-CH_2CH_2-$ | b) 1,4735 | 76,4 |

**Ansprüche**

1. Verfahren zur Herstellung von N-Alkoxycarbonyl- oder N-Alkoxythiocarbonylamidinen der Formel

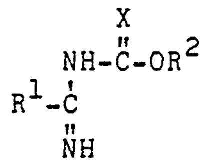
(I)

worin $R^1$ ein Wasserstoffatom, einen aliphatischen, cycloaliphatischen oder araliphatischen Rest bedeutet, $R^2$ einen aliphatischen Rest bezeichnet und X für ein Sauerstoffatom oder ein Schwefelatom steht, dadurch gekennzeichnet, daß man Amidine der Formel

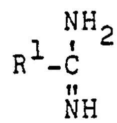
(II)

worin $R^1$ die vorgenannte Bedeutung besitzt, oder ihre Amidinsalze mit Estern der Chlorkohlensäure oder Chlorthiokohlensäure der Formel

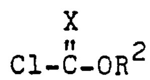
(III)

worin $R^2$ und X die vorgenannte Bedeutung besitzen, in Gegenwart eines anorganischen Säureakzeptors und eines 2-phasigen, wäßrig-organischen Lösungsmittelgemisches bei einer Temperatur von 0 bis 60 °C umsetzt.

2. N-Alkoxycarbonyl- und N-Alkoxythiocarbonylamidine der Formel

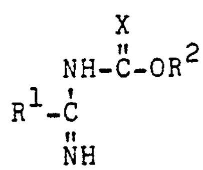
(I)

worin $R^1$ ein Wasserstoffatom, einen aliphatischen, cycloaliphatischen oder araliphatischen Rest bedeutet, $R^2$ einen aliphatischen Rest bezeichnet und X für ein Sauerstoffatom oder ein Schwefelatom steht.

**Claims**

1. A process for the preparation of an N-alkoxycarbonyl- or N-alkoxythiocarbonyl-amidine of the formula

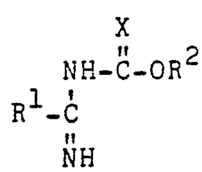
(I)

where $R^1$ is hydrogen or an aliphatic, cycloaliphatic or araliphatic radical, $R^2$ is an aliphatic radical and X is oxygen or sulfur, wherein an amidine of the formula

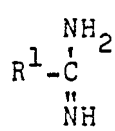
(II)

where $R^1$ has the above meanings, or an amidine salt thereof, is reacted with an ester of chlorocarbonic acid or chlorothiocarbonic acid of the formula

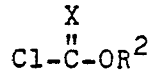

(III)

where $R^2$ and X have the above meanings, in the presence of an inorganic acid acceptor and a 2-phase aqueous/organic solvent mixture at from 0 to 60 °C.

2. An N-alkoxycarbonyl- or N-alkoxythiocarbonylamidine of the formula

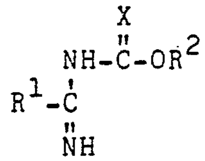

(I)

where $R^1$ is hydrogen or an aliphatic, cycloaliphatic or araliphatic radical, $R^2$ is an aliphatic radical and X is oxygen or sulfur.

**Revendications**

1. Procédé de préparation de N-alcoxycarbonyl- et de N-alcoxythiocarbonyl-amidines de la formule

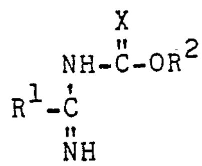

(I)

dans laquelle $R^1$ désigne un atome d'hydrogène ou un reste aliphatique, cycloaliphatique ou araliphatique, $R^2$ un reste aliphatique et X un atome d'oxygène ou un atome de soufre, caractérisé en ce que l'on fait réagir des amidines de la formule

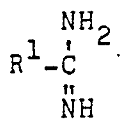

(II)

dans laquelle $R^1$ possède la signification définie, ou un sel d'une telle amidine, à une température comprise entre 0 et 60 °C et en présence d'un fixateur d'acide inorganique, dans un mélange solvant aqueux-organique à deux phases avec des esters de l'acide chloro-carbonique ou de l'acide chlorothiocarbonique de la formule

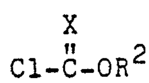

(III)

2. N-Alcoxycarbonyl- et N-alcoxythiocarbonyl-amidines de la formule

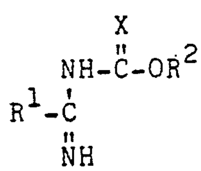

(I)

dans laquelle $R^1$ désigne un atome d'hydrogène ou un reste aliphatique, cycloaliphatique ou araliphatique, $R^2$ un reste aliphatique et X un atome d'oxygène ou de soufre.